# EUROPEAN PATENT APPLICATION

(11) **EP 4 767 979 A1**
(43) Date of publication of application: **01.07.2026**
(21) Application number: 24315605.6
(22) Date of filing: 31.12.2024
(51) Int. Cl.: A61B 34/20, A61C 1/08, A61B 90/00, A61C 9/00

(54) **A DENTAL SPLINT AND METHOD OF MANUFACTURE**

(71) Applicant: Digicuto, 13008 Marseille (FR)
(72) Inventor: Matthieu Jean Raymond Bouisson, 13008 Marseille (FR); Maxence Pinta, 13008 Marseille (FR); Aldéric Gasse, 13008 Marseille (FR); Galip Gurel, 13008 Marseille (FR); Stefan Alexandre Sylvain David Koubi, 13008 Marseille (FR); Karim Tourbah, 13008 Marseille (FR)
(74) Representative: Slingsby Partners LLP

(57) **Abstract**

A dental splint for providing a positional reference with respect to dentition of a subject. The splint comprising a frame having a first clamping element and a second clamping element movable with respect to the first clamping element to mechanically grip a first tooth of the subject; and a first extension arm rigidly attached to the frame and extending from the frame for supporting an optical reference unit at a location outside the oral cavity of the subject when the frame is gripping the first tooth. There is also provided a method for providing a positional reference with respect to dentition of a subject. The method comprising causing a dental splint as claimed in any preceding claim to grip a tooth of the subject; and imaging an optical reference unit carried by the or each extension arm of the dental splint.

## Description

### FIELD OF THE INVENTION

This invention relates to robotically assisted or executed dentistry. For example, application of veneers and insertion of dental implants.

### BACKGROUND

In everyday life, dental and medical problems are unfortunately commonplace. And if patients cannot receive appropriate treatment in time, these dental and medical problems can be extremely serious and painful, affecting their quality of life and health. In order to treat patients more quickly, there are increasing plans to use robots to assist practitioners in carrying out dental or medical procedures. Indeed, thanks to their controllable movements, robots can not only improve the efficiency of dental or medical procedures, but also the efficiency of treatment by avoiding or limiting human errors.

The use of robots in performing dental procedures has also become increasingly viable in recent decades due to advances in both robot technology and material science for dentistry. The use of robots in such procedures relies on providing a stable reference frame within which the robot operates. Existing systems often comprise attaching markers for this reference frame which are fixed relative to the subject in some way. There are many existing methods for determining these reference points for facilitating robot control. However, it can often be difficult to determine precise reference points relative to a subject's body, especially if the subject is prone to moving. Similarly, maintaining the same reference frame across multiple appointments can be challenging due to the removal and re-attachment of such reference points.

A particular field in which consistent and stable reference points are important is dentistry and other facial procedures. For example, such procedures may comprise, but are not limited to, attaching veneers, crowns, or implants, but also procedures such as orthodontic treatment, maxillofacial surgery, and facial surgery in general.

It is therefore desirable to develop an apparatus for providing stable and consistent reference points for robot assisted dental and facial procedures.

### SUMMARY OF THE INVENTION

According to one aspect there is provided a dental splint for providing a positional reference with respect to dentition of a subject, the splint comprising: a frame having a first clamping element and a second clamping element movable with respect to the first clamping element to mechanically grip a first tooth of the subject; and a first extension arm rigidly attached to the frame and extending from the frame for supporting a reference unit at a location outside the oral cavity of the subject when the frame is gripping the first tooth.

In an embodiment, the first clamping element may be integral with the second clamping element, and the first and second clamping elements may be flexibly interconnected such that the first and second clamping elements can be urged towards each other to grip the first tooth.

In an embodiment, the splint may comprise a locking piece sized to be engageable against the first and second clamping elements to urge the first and second clamping elements into contact with the first tooth.

In an embodiment, the locking piece may define a channel having side walls that can be engaged against the first and second clamping elements to urge the first and second clamping elements into contact with the first tooth.

In an embodiment, the locking piece may be configured to releasably latch to the first and second clamping elements for holding it in engagement with the first and second clamping elements.

In an embodiment, the locking piece may be non-integral with the clamping elements.

In an embodiment, the locking piece may comprise a shoulder configured to engage a complementary protrusion located elsewhere on the dental splint for retaining the locking piece on the dental splint when not latched to the first and second clamping elements.

In an embodiment, the frame may have a third clamping element and a fourth clamping element movable with respect to the third clamping element to mechanically grip a second tooth of the subject, the second tooth being on the opposite quadrant of the same jaw to the first tooth.

In an embodiment, the splint may comprise a second locking piece sized to be engageable against the third and fourth clamping elements to urge the third and fourth clamping elements into contact with the second tooth.

In an embodiment, the first extension arm may be attached to the frame adjacent the first and second clamping elements and the splint may comprise a second extension arm for supporting the reference unit at a location outside the oral cavity of the subject when the frame is gripping the second tooth, the second extension arm being rigidly attached to the frame adjacent the third and fourth clamping elements.

In an embodiment, one of the first and second clamping elements may have a leading end and a trailing end, the trailing end being cupped so as to engage the occlusal surface of the first tooth and thereby limit insertion of the first tooth between the first and second clamping elements; one of the third and fourth clamping elements may have a further leading end and a further trailing end, the further trailing end being cupped so as to engage the occlusal surface of the second tooth and thereby limit insertion of the second tooth between the third and fourth clamping elements; the leading end and the further leading end may lie in a common planar zone; and the frame may comprise a bridge extending between (i) the first and second and (ii) the third and fourth clamping elements, the bridge being located no closer to the cupped regions of the first to fourth clamping elements than the common planar zone.

In an embodiment, the or each extension arm may carry a receptacle for releasably receiving the reference unit.

In an embodiment, the or each extension arm may carry the reference unit.

In an embodiment, the reference unit may be an optical reference unit comprising a plurality of optical reference sites.

In an embodiment, each optical reference site may comprise a recess for releasably receiving a reflector and a key surface shaped for fixing the position of the reflector relative to the frame.

In an embodiment, the optical reference unit may have at least three optical reference sites, the optical reference sites being located so as to be coplanar and not all collinear.

In an embodiment, the reference unit may be an inertial reference unit or a magnetic reference unit.

In an embodiment, the splint may be arranged so that when the first tooth or the second tooth is a molar or premolar of the subject, the splint does not occlude the facial surfaces of the incisors of the same dental arch as the first tooth.

In an embodiment, the first and second clamping elements, and the third and fourth clamping elements may be shaped to match the contours of molars and/or premolars of a subject.

In an embodiment, the first tooth or the second tooth may be of a first dental arch of the subject and the splint may comprise a jaw plate for engaging the other dental arch of the subject, the jaw plate being releasably attachable to the frame in a position to engage the other dental arch and thereby restrict motion of the jaws of the subject towards each other.

In an embodiment, either the frame or the jaw plate may comprise a tongue restraint for restraining the tongue of the subject.

In an embodiment, either the frame or the jaw plate may comprise a formation for holding a suction tube in the subject's oral cavity.

According to another aspect there is provided a method for providing a positional reference with respect to dentition of a subject, the method comprising: causing a dental splint as claimed in any preceding claim to grip a tooth of the subject; and imaging a reference unit carried by the or each extension arm of the dental splint.

In an embodiment, the method may comprise: forming the first and second clamping elements to match the contours of the first tooth of the subject or forming the third and fourth clamping elements to match the contours of the second tooth of the subject.

In an embodiment, the method may comprise: scanning the first tooth or second tooth so as to form a set of three-dimensional data representing the shape of the first tooth or second tooth; and forming the first and second clamping elements or the third and fourth clamping elements in dependence on the set of three-dimensional data.

In an embodiment, the method may comprise forming the first and second clamping elements or the third and fourth clamping element by additive manufacturing.

According to another aspect there is provided a dental splint comprising: an arcuate jaw clamp extending around an arc of greater than 120 degrees and having a first recess at one end thereof and a second recess at the other end thereof, each recess having at least one side wall for contacting a lateral surface of at least one tooth of a subject and a basal surface extending transversely to the side wall for contacting occlusal surfaces of at least one tooth in the recess for limiting the insertion of at least said tooth into the recess in a first direction; wherein both the basal surfaces lie on a common basal plane and the jaw clamp has a bridge extending between the first recess and the second recess, a central region of the bridge extending, on the convex side of the arc, no further in a direction opposite to the first direction than the basal plane.

In an embodiment, each recess may comprise a first clamping element and a second clamping element, the clamping elements of each recess being moveable with respect to each other to mechanically grip a tooth of the subject located in the respective recess.

In an embodiment, the splint may comprise at least one extension arm rigidly attached to the arcuate jaw clamp and extending from the arcuate jaw clamp for supporting a reference unit at a location outside the oral cavity of the subject when the arcuate jaw clamp is engaged with one or more teeth in the recesses.

In an embodiment, the extension arm may carry a receptacle for releasably receiving the reference unit.

In an embodiment, the extension arm may carry the reference unit.

According to another aspect there is provided a dental splint comprising: an arcuate jaw clamp extending around an arc of greater than 120 degrees and having a first recess at one end thereof for receiving a first molar or premolar of a subject, and a second recess at the other end thereof for receiving a second molar or premolar of the subject, the first and second molars or premolars being in the same dental arch but different quadrants thereof; the jaw clamp comprising a bridge extending between the first recess and the second recess and shaped such that when the first recess receives the first molar or premolar and the second recess receives the second molar or premolar, the bridge does not occlude the facial surfaces of the incisors of the said dental arch.

In an embodiment, each recess may comprise a first clamping element and a second clamping element, the clamping elements of each recess being moveable with respect to each other to mechanically grip a tooth of the subject located in the respective recess.

In an embodiment, the splint may comprise at least one extension arm rigidly attached to the arcuate jaw clamp and extending from the arcuate jaw clamp for supporting a reference unit at a location outside the oral cavity of the subject when the arcuate jaw clamp is engaged with one or more teeth in the recesses.

In an embodiment, the extension arm may carry a receptacle for releasably receiving the reference unit.

In an embodiment, the extension arm may carry the reference unit.

### BRIEF DESCRIPTION OF THE FIGURES

The present invention will now be described by way of example with reference to the accompanying drawings. In the drawings:
Figure 1 shows an example dental splint.
Figure 2 shows an example dental splint viewed from above.
Figure 3 shows an example dental splint viewed from the back.
Figure 4 shows a perspective view of an example dental splint.
Figure 5 shows a perspective view and a side view of an example locking piece.
Figure 6 shows a perspective view of an example locking piece engaged with clamping elements.
Figure 7 shows a plan view of an example jaw plate for engaging the other dental arch of the subject.
Figure 8 shows a perspective view of an example dental splint.
Figure 9 shows an example dental splint.
Figure 10 shows a perspective view of an example jaw plate.
Figure 11 shows a perspective view from the side of an example jaw plate comprising an end region/cross-section near a tongue restraint.
Figure 12 shows a front view of an example jaw plate.
Figure 13 shows an example dental splint.
Figure 14 shows a rear view of an example dental splint.
Figure 15 shows a perspective view of an example dental splint.
Figure 16 shows a perspective view of an example embodiment of a dental splint viewed from the front.
Figure 17 shows a perspective view of an example embodiment of a dental splint viewed from below.

### DETAILED DESCRIPTION OF THE INVENTION

There is provided herein a dental splint for providing a positional reference with respect to dentition of a subject. Such a splint may be used in dental procedures which make use of a robot controlled or manipulated dentistry tool. For example, tools for milling the surface of teeth during a process of applying veneers or other cosmetic and/or functional dental prostheses. Non-limiting examples other than veneers include crowns and implants.

The proposed dental splint could be applied to other types of treatment, such as crown or implant, but also such as orthodontic treatment, maxillofacial surgery, and facial surgery in general. In particular, the system enables reference markers to be connected to a subject in a stable way, since they are connected to a "hard" part of the subject's anatomy, the teeth, without being invasive. That is, the reference markers in this way are connected indirectly to the jawbone, for example, or to the skull bone via the teeth.

As mentioned above, applying veneers is just one example of a dental procedure in which such a dental splint may be used. During a conventional treatment process, there is typically a visit which consists of milling the patient's teeth. There is then a further visit which consists of fitting the dental prostheses that will have been made since the previous visit where the milling took place. A robotic tool may be used to provide the milling. In order to achieve the described process, the spatial position and orientation of the tooth or teeth to be milled needs to be precisely known in relation to the spatial position and orientation of the robotic tool. This allows a controller of the robot to mill the teeth as required for the veneers.

The spatial alignment (in position and orientation) may be achieved by use of a detecting system directed towards the subject and one or more markers fixed relative to the subject's teeth. There may also be markers fixed to the robot. The detecting system can then be used to detect the markers and use them as reference points for knowing the relative position of the subject's teeth to the tool. A processor can detect the markers in an image captured by the detecting system. The processor can estimate the relative position and orientation of the teeth and the robot in dependence on the relative positions of the markers in an image captured by the detecting system. The teeth may be provided with multiple markers, which may be arranged to be non-collinear. This can assist in detecting the orientation of the teeth. The robot may be provided with multiple markers, which may be arranged to be non-collinear. This can assist in detecting the orientation of the robot. In each case the distance between the markers as observed by the detecting system can be used to estimate the direction and distance of the teeth/robot with respect to the detecting system. The detecting system may comprise a stereoscopic camera which can detect optical markers. Alternatively, the dental splint could be used alongside another optical tracking system using a laser as the detection system rather than a camera. In another alternative, the dental splint could be used alongside an inertial tracking system, in which case the detected inertial information may be provided to the robot or an intermediate control entity. In another alternative, the dental splint could be used alongside a magnetic tracking system, in which case the dental splint could be linked to an arm attached to the robot.

Existing ways of fixing one or more optical markers to the subject comprise removably fixing one or more markers to the subject's other teeth, i.e. those which are not undergoing work. For example, typically a stick-like item having an optical marker on one end may be fixed across the face of one or more other teeth of the subject, e.g. the molars, with the optical marker end orientated towards the front of the mouth. The object and optical marker may therefore be temporarily attached with adhesive or cement to the subject's teeth so that the marker is fixed with respect to the subject's teeth. However, this is not always a secure bond. The bond may have some elasticity which permits the marker to move relative to the teeth. Consequently, the optical marker may not be as positionally accurate as needed and may be accidentally dislodged.

It has been recognised that a more stable, secure, and positionally accurate way of determining the location of a subject's teeth relative to a computer-controlled tool is needed.

There is provided herein a dental splint for providing a positional reference with respect to dentition of a subject. Accordingly, the splint may be rigidly fixed in position relative to the subject's teeth and provide a means for securing one or more reference points.

The proposed dental splint will now be described in reference to the example embodiment shown in the figures. It should be understood that the dental splint is not limited to the specific embodiment shown in any one of the figures. It should also be understood that when implemented, different features presented alongside the content of different figures may be combined in any combination as defined by the claims.

The proposed system is designed to treat the teeth of the maxilla and mandible. In this context, the frame can be engaged with one or more teeth of the upper jaw if the treatment is performed on one or more teeth of this jaw, or the frame can be engaged with one or more teeth of the lower jaw if the treatment is performed on one or more teeth of this jaw. That is, the frame is engaged with the jaw being treated. Thus, in a third implementation, the splint may be engaged simultaneously with the dental arch of the upper jaw and the dental arch of the lower jaw.

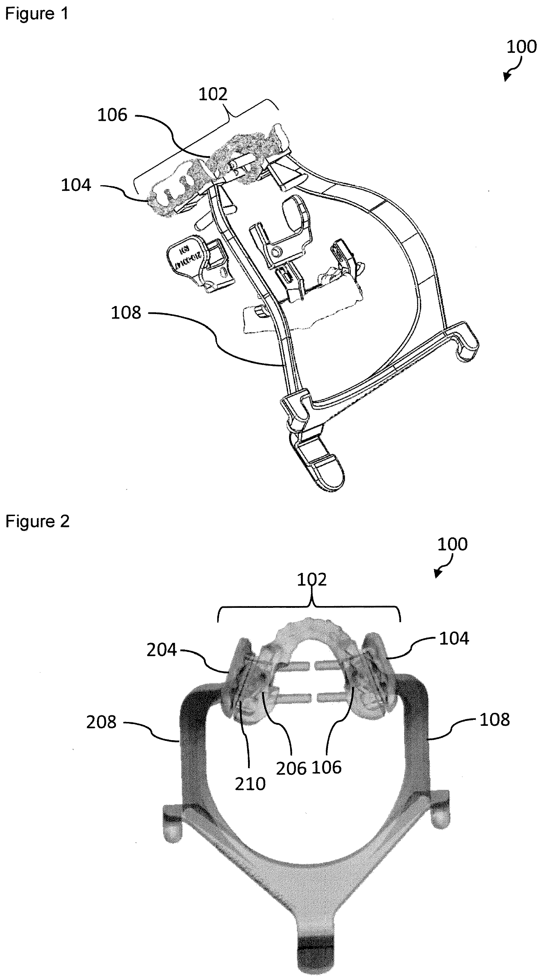

Figure 1 shows an example embodiment of the dental splint 100. The splint 100 comprises a frame 102 having a first clamping element 104 and a second clamping element 106. The second clamping element 106 is movable with respect to the first clamping element 104 to mechanically grip a first tooth of the subject. That is, the frame provides two halves of a gripping portion of the splint which can engage one or more neighbouring teeth of the subject. The frame 102, as shown in figure 1, is configured to engage with the upper dental arch of the subject. It should be understood that the first and second clamping elements need only engage one side of the dental arch. A dental arch is the teeth of the upper jaw or the teeth of the lower jaw.

The splint 100 also comprises a first extension arm 108 rigidly attached to the frame 102 and extending from the frame 102 for supporting a reference unit at a location outside the oral cavity of the subject when the frame is gripping the first tooth. The extension arm may be rigidly attached to the frame at one end and extend in a direction perpendicular to the plane of the biting surface of the upper dental arch. The extension arm may be shaped to exit the oral cavity of the subject and clear the subject's lower jaw. The reference unit may be any structure which may be detected and used to provide a reference point for relative positioning of the splint, and therefore the teeth of the subject, relative to a robotically manipulated dental tool.

In an embodiment the splint structures described, such as the frame, may be implemented on both dental arches at once. Any reference herein to upward or downward directions relative to teeth etc. are made in reference to the example dental splint shown in the figures and are not intended to limit the use of the splint to any single dental arch/jaw configuration. Further, description of the orientation of the dental splint is based on the example dental splint shown in the figures orientated while in use and looking at the face of the subject. Though it should be understood that when in use the example dental splint may be fixed to a subject while the subject is in a reclining or supine position.

Figure 2 shows an example embodiment of the splint 100 viewed from above. The frame 102 is located at the top of the splint 100 and the extension arm 108 extends towards the bottom of the splint 100 (into the plane of the page). The first clamping element 104 may be integral with the second clamping element 106. That is, the first and second clamping elements may be formed as different parts of a single structure.

The first and second clamping elements may be flexibly interconnected such that the first and second clamping elements can be urged towards each other to grip the first tooth. Figure 2 shows how the first and second clamping elements may be interconnected along a portion of the frame below the biting surface of the subject's upper teeth. This join 210 may comprise a single connection or multiple smaller connections. The number of connections between the first and second clamping element may depend on the desired flexibility. The thickness of the connection(s) between the first and second clamping element may depend on the desired flexibility.

The length of the connection(s) between the first and second clamping element may depend on the desired flexibility.

The frame 102 may have a third clamping element 204 and a fourth clamping element 206. The fourth clamping element 206 may be movable with respect to the third clamping element 204 to mechanically grip a second tooth of the subject. The second tooth may be located on the opposite quadrant of the same jaw to the first tooth.

The first extension arm 108 may be attached to the frame adjacent the first clamping element 104 and the second clamping element 106 and the splint 100 may comprise a second extension arm 208 for supporting the reference unit at a location outside the oral cavity of the subject when the frame is gripping the second tooth, the second extension arm being rigidly attached to the frame adjacent the third clamping element 204 and the fourth clamping element 206. That is, where there are two extension arms, they may each be attached to the frame 102 next to one of the two sets of clamping elements as shown in figure 2.

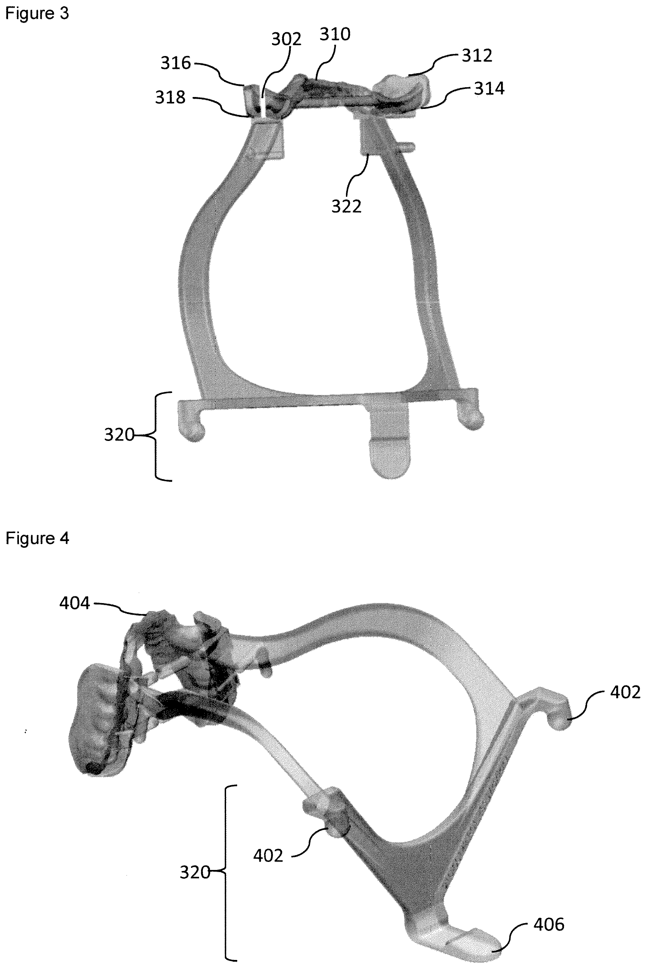

Figure 3 shows an example embodiment of the dental splint 100 as viewed from the back. In this figure the dental splint 100 is orientated such that the subject's molars are in front of the incisors and the extension arm extends downwards and away from the subject's lower jaw (into the plane of the page). The extension arm may curve backwards around the lower jaw towards the subject. The extension arm may terminate in a structure 320 for supporting a reference unit.

There may be a gap or channel or slot defined between the first and second clamping elements and between the third and fourth clamping elements. Figure 3 shows this slot 302, which allows for the third and fourth clamping elements to be urged together to grip around the one or more teeth of the subject.

One of the first and second clamping elements 104,106 may have a leading end 312 and a trailing end 314. The trailing end 314 may be cupped so as to engage the occlusal surface of the first tooth and thereby limit insertion of the first tooth between the first and second clamping elements. That is, the edge located towards the biting surface of the tooth may be cupped. The cupped portions may surround and hold a portion of the biting surface of at least the first tooth and prevent it from passing between the griping surfaces of the clamping elements.

One of the third and fourth clamping elements 204, 206 may have a further leading end 316 and a further trailing end 318. The further trailing end 318 may be cupped so as to engage the occlusal surface of the second tooth and thereby limit insertion of the second tooth between the third and fourth clamping elements. Similarly, the edge of the third and fourth clamping elements located towards the biting surface of the tooth may be cupped. The cupped portion may surround and hold the biting surface of at least the second tooth and prevent it from passing between the griping surfaces of the clamping elements.

The leading end and the further leading end may lie in a common planar zone. That is, the top edges of the clamping elements closest to the gums of the dental arch lie in a zone around a common plane. The common planar zone is therefore approximately parallel to the plane of the dental arch.

The frame may comprise a bridge 310 extending between the first and second, and the third and fourth clamping elements. The bridge 310 therefore joins together the two pairs of clamping elements located at the ends of the dental arch. The bridge may be located no closer to the cupped regions of the first to fourth clamping elements than the common planar zone. That is, for example, when considering the dental clamp positioned on an upper jaw, the bridge may be located above the edges of the clamping elements inside the dental arch. This may coincide with the inside gum line of the molars and premolars. For example, the bridge may be shaped to cover the flat portion of the hard palate and/or the arched part of the hard palate behind the front incisors. As such, it may span part of the roof of the subject's mouth. Alternatively, for implementing the dental splint for the lower jaw, it may sit under the subject's tongue.

Figure 4 shows a perspective view of the dental splint. The splint is illustrated as viewed from below at an approximate 45-degree angle towards left from centre. The frame is towards the background while the structure 320 for supporting the reference unit is in the foreground. In this example, the two are connected by two extensions arms. The or each extension arm may carry a receptacle 402 for releasably receiving the reference unit. In this example, the receptacle is illustrated as a spherical ended protrusion 402 which is angled such that a reference unit may be held behind it. It should be understood that the receptacle could take many forms to provide this function, for example, a tab or hook. The tab or hook could be shaped to fit a corresponding notch or groove in the reference unit. The receptacle could instead be a notch or groove for receiving a tab or hook on the reference unit.

The receptacle 402 may form part of the structure 320 for receiving and securing the reference unit. For example, in figure 4 the structure 320 is a triangular structure or Y shaped yoke at the ends of a pair of extension arms. The receptacle 402 may be located on the or an extension arm which then may be configured to continue and terminate in a further receptacle or a clip 406. Where there are a plurality of extension arms, the extension arms may meet at the end and a single further receptacle or a clip 406 may be located in the area of intersection. Therefore, the or each extension arm may carry the reference unit.

The reference unit may have a plurality of reference sites. That is, for example, in the case of an optical reference unit, the optical reference unit may comprise multiple sites comprising individually optically detectable points which may be used together to provide a reference frame for relative positioning of the splint. Each optical reference site may comprise a recess for releasably receiving a reflector and a key surface shaped for fixing the position of the reflector relative to the splint frame. The reference unit may have at least three optical reference sites, the optical reference sites being located so as to be coplanar and not all collinear.

The splint may be arranged so that when the first tooth or the second tooth is a molar or premolar of the subject, the splint does not occlude the facial surfaces of the incisors of the same dental arch as the first tooth. A portion of the frame may be shaped to fit the inside concaved surface of the dental arch. The portion of the frame shaped to fit the inside concaved surface of the subject's dental arch includes an apex 404. The apex 404 is shaped to sit behind the front teeth or against part of the palate of the subject. This way the surfaces of the front teeth are not obstructed and may be accessed for dental procedures. The first and second clamping elements, and the third and fourth clamping elements may be shaped to match the contours of molars and/or premolars of the subject. This allows for a precise and well-defined orientation of the splint. As the gripping elements match the shape of the teeth they are gripping, there is little to no sliding or shifting of the gripping elements possible across the surface of the teeth once gripped.

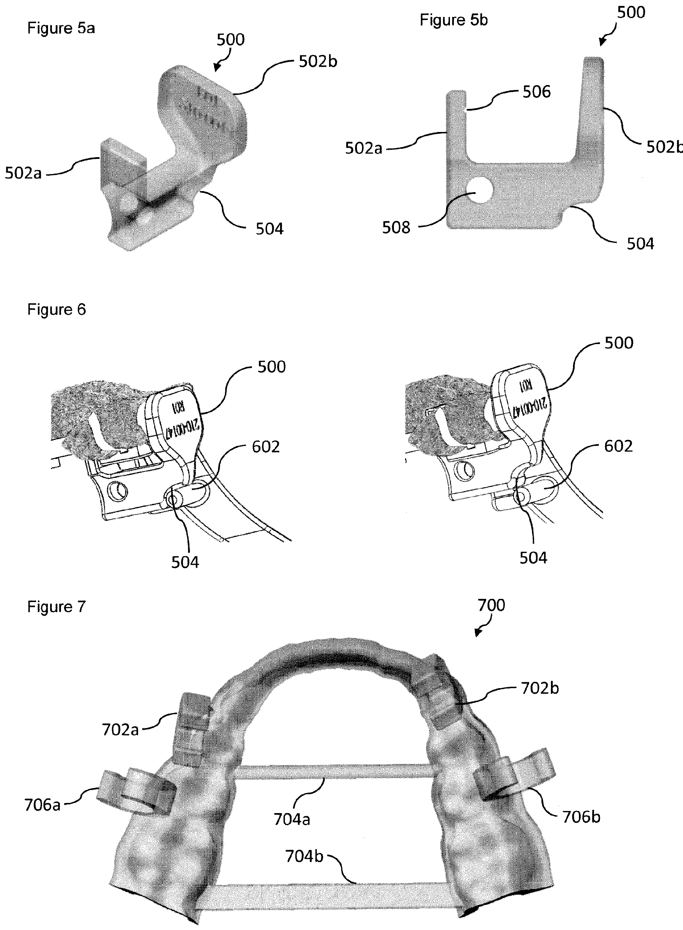

The splint 100 may comprise a locking piece 500 sized to be engageable against the first and second clamping elements 104 and 106 to urge the first and second clamping elements into contact with the first tooth. Figure 5a shows a perspective view of an example locking piece 500. The locking piece may be sized to fit over the first and second clamping elements and push them together. The locking piece may define a channel having side walls 502a, 502b that can be engaged against the first and second clamping elements to urge the first and second clamping elements into contact with the first tooth. That is, the locking piece 500 may be U-shaped and configured to slide over the first and second clamping elements so as to force them together to grip the first tooth. The locking piece may be configured to releasably latch to the first and second clamping elements for holding it in engagement with the first and second clamping elements. The locking piece may be non-integral with the clamping elements. That is, the locking piece may be a separate item from the clamping elements it is configured to engage.

The splint may comprise a second locking piece sized to be engageable against the third and fourth clamping elements to urge the third and fourth clamping elements into contact with the second tooth. The second locking piece may be identical to the first locking piece. The second locking piece may be a mirror image of the first locking piece. That is, the second locking piece may have the same structural features as the first locking piece, but these features may be arranged to engage the opposite side of the same dental arch, i.e. the third and fourth clamping elements, thus requiring those structural features to be reversed or rotated 180 degrees.

The side walls 502a and 502b may not be the same size or shape as each other. For example, a first side wall 502a may be smaller than a second side wall 502b. The internal surfaces of the side walls, the surfaces within the channel, may be shaped to cooperate with the outside surfaces of the first and second clamping element or the third and fourth clamping element respectively.

Figure 5b shows a side view of the locking piece 500. Side wall 502a may be shorter than side wall 502b. This may be because side wall 502a lies inside the dental arch of the subject, whereas side wall 502b lies outside the dental arch of the subject. Therefore, the second side wall 502b has a greater amount of space over the gums which remains in the same plane as the facial surface of the teeth/tooth being gripped. However, the first side wall 502a may have less space due to the palate of the subject, which may extend at an angle towards the roof of the mouth and outward from approximately the base of the teeth/tooth being gripped. It should be understood that should the dental splint be configured for engaging a different part of or whole dental arch, the walls of the locking piece may be sized accordingly to account for available depth and access to the necessary teeth/tooth.

The locking piece may be engaged against the first and second clamping elements in two configurations.

In a first configuration, the locking piece is in an unlocked or relaxed configuration. In the unlocked or relaxed configuration, the locking piece is loosely engaged against the side walls of the first and second clamping elements or against the side walls of the third and fourth elements. A respective locking piece is loosely engaged with each pair of clamping elements.

In a second configuration, the locking piece is in a locked or tight configuration. In the locked or tight configuration, the locking piece is latched against the side walls of the first and second clamping elements or against the side walls of the third and fourth clamping elements. A respective locking piece is latched to each pair of clamping elements.

The locking piece may comprise a shoulder 504.

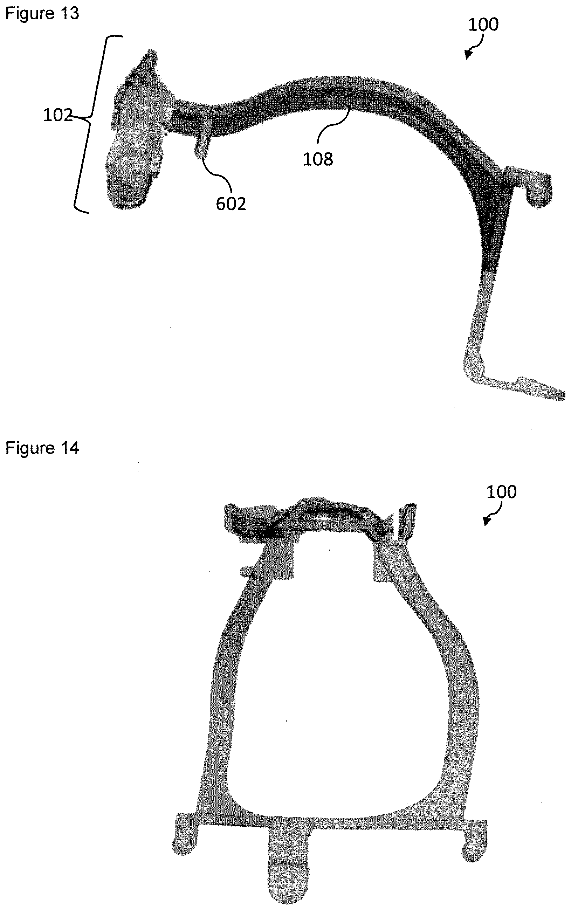

In the unlocked or relaxed configuration, the shoulder 504 may rest on a post or complementary shaped protrusion 602 as shown in Figure 6a. In this way, in the unlocked or relaxed configuration the locking piece may be retained by the corresponding protrusion. The corresponding protrusion may be located on another part of the dental splint, for example the complementary protrusion may be located on an extension arm as shown in Figure 6 and in Figure 13. The protrusion may retain the locking piece on the dental splint during placement of the dental splint on the subject's jaw.

In the locked or tight configuration, the shoulder 504 and the protrusion 602 are no longer in contact, as shown in Figure 6b.

When the dental splint is placed on the subject's jaw, the locking piece can be slid, over the side walls of the first and second clamping elements towards the leading end 312 or over the side walls of the third and fourth clamping elements towards the further leading end 316, to move from the unlocked configuration to the locked configuration. In the locked configuration, the side walls of the first and second clamping elements clamp the first tooth or the side walls of the third and fourth clamping elements clamp the second tooth. In the locked configuration, the dental splint is positioned in a fixed and stable manner on the subject's jaw.

When the dental splint is removed from the subject's jaw, the locking piece can be slid, over the side walls of the first and second clamping elements or over the side walls of the third and fourth clamping elements, towards the protrusion 602 until the shoulder 504 of the locking piece rests on the protrusion 602, to move from the locked configuration to the unlocked configuration. In the unlocked configuration, the side walls of the first and second clamping elements release the first tooth or the side walls of the third and fourth clamping elements release the second tooth. In the unlocked configuration, the dental splint is loosely positioned on the subject's jaw.

The locking piece may comprise a notch or ridge 506 for engaging a corresponding ridge or notch respectively on the first or second clamping element. In this way the locking piece may be configured to releasably latch to the first and second clamping elements for holding it in engagement with the first and second clamping elements. This may be achieved by any number of similarly shaped cooperating portions of the clamping elements and locking piece for releasably securing them together. Additionally, or alternatively, these cooperating portions, comprising for example a notch and ridge, provide haptic feedback while fitting the dental splint. That is, the movement of the corresponding notch and ridge over each other can provide haptic feedback to the person assembling the dental splint to indicate that the locking piece is sufficiently covering the clamping elements. As such, the haptic feedback may indicate that the locking piece does not need to be pushed any further over the clamping elements or that the locking piece has reached a minimum amount of overlap with the clamping elements. In this way in can be assured that the locking piece will not accidentally slip off of the clamping elements.

The proposed system is specifically designed to be easy to fit to and remove from the subject's jaw. In particular, the splint is designed such that it is not necessary to use an element independent of the splint in order to be able to install it in the subject's mouth. That is, the elements constituting the dental splint as such are alone sufficient for enabling the fitting. Similarly, it is not intended to be necessary to use a tool to remove the dental splint from the subject's mouth, although mechanisms to facilitate removal are proposed for added safety and convenience.

The locking piece may additionally comprise a hole 508. The hole 508 is provided so that a small tool independent of the dental splint may be inserted to facilitate removal of the locking piece from the splint and the subject's mouth. Although, the locking piece is designed to be easily removed, the tool may be used in the hole 508 as a last resort to ensure the splint can be removed in a safe and controlled manner.

Figure 7 shows a plan view of an example jaw plate 700 for engaging the other dental arch of the subject which is not being treated. The jaw plate 700 is shown from above when configured to engage the lower dental arch of the subject such that the relief of the impressions of the occlusal surfaces can be seen. It should be understood that the relief impressions may not always be visible in this manner as the opposite side of the jaw plate to the teeth of the subject may be moulded to any desired shape. That is, the first tooth or the second tooth may be of a first dental arch of the subject and the splint may comprise a jaw plate for engaging the other dental arch of the subject.

The jaw plate may be releasably attachable to the frame in a position to engage the other dental arch and thereby restrict motion of the jaws of the subject towards each other. For example, the jaw plate 700 may comprise one or more formations 702a and 702b for engaging one or more corresponding portions of the frame 102 and preventing the two parts from moving closer together. An example of a corresponding portion of the frame is shown in figure 3, whereby an elongate protrusion 322 extends from at least one extension arm to engage the formation 702.

Further, either the frame 102 or the jaw plate 700 may comprise a tongue restraint 704a and/or 704b for restraining the tongue of the subject. Two examples of such tongue restraints are shown in figure 7. The one or more restraints may also act as a support to provide stability to the structure of the jaw plate 700.

Additionally, either the frame 102 or the jaw plate 700 may comprise a formation 706 for holding a suction tube in the subject's oral cavity. This allows for a suction tube to be positioned for removing excess saliva of the subject throughout the dental procedure without always needing to be held by the dentist or assistant. The suction tube may also be easily repositioned with regard to location inside the subject's mouth and its angle. A plurality of formations 706a and 706b may provide different angles for the suction tube so as to remove the saliva in the most efficient and comfortable position.

The jaw plate 700 as shown in figure 7 engages the whole of the dental arch not being worked on. However, in some embodiments the jaw plate may only engage a portion of the opposite dental arch. For example, the jaw plate may only engage one or more molars towards the back of the opposite dental arch. The jaw plate in this configuration may take the shape of a wedge, which is deeper toward the front of the subject's mouth than it is towards the back of the subject's mouth. In this way the shape of the jaw plate may mirror the angle between the two dental arches and set a minimum angle at which they can be spaced apart. This enables the subject's mouth to remain at this open angle without the subject being required to hold this position.

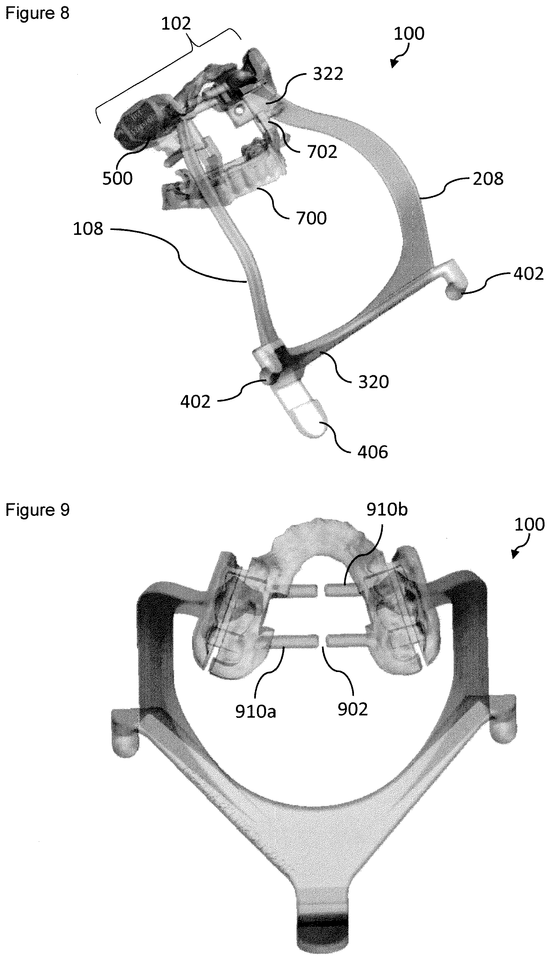

Figure 8 shows a perspective view of an example dental splint 100 comprising the frame 102, extension arm(s) 108, 208 for supporting a reference unit, locking piece 500, and jaw plate 700 in an assembled configuration. In this example the jaw plate 700 engages the frame 102 between two pairs of corresponding formations 702 and elongate protrusions 322.

As described above, on the outside of the molars there may be a clamping element or wall and on the inside of the molars there may be another clamping element or wall. Those walls may have a gap between them. The frame comprising the walls may be placed inside the subject's mouth with the channel between those walls going over the molars and the base of the channel seated on the occlusal surfaces, top or bottom biting surfaces, of the molars. The locking pieces or clips, which comprise two essentially U-shaped clips, may then be pushed over the two wing-like walls which forces the walls together to mechanically grip the teeth. That is, the locking pieces go over the outside of the wing-like walls and force them together into contact with the teeth. The locking pieces may be put in place by pushing them on. The locking pieces may clip into place. The locking pieces may be held in place by friction.

There may be two extension arms fixed to the frame and at the end of those there may be an interface for supporting a referent unit. The interface may allow for the reference unit to be clipped onto the splint. The reference unit may have some markers on it that a detecting system can be used to detect. The purpose of the one or more extension arms is to extend out of the mouth so that the reference unit may be located outside the mouth and away from where the robot is working. Therefore, the reference unit is not getting in the way of the robot.

When markers are optical markers, a camera can be used to image optical markers and there may be optical markers on both the robot and associated with the subject. Typically, in this way the optical reference unit is a plate with optical markers on it. The plate may be of any shape suitable for attaching one or more optical markers. In the example shown in figure 8, the optical reference unit is a plate sized to fit within the receptacles 402 and the further receptacle or clip 406. The plate may sit within the receptacles 402 and the further receptacle or clip 406 and sit flush against the surface of the structure 320 which may be a triangular structure or Y shaped yoke. By imaging the optical markers on the subject and the robot it is possible to determine the spatial relationship between the subject and the robot.

The jaw plate 700 is a removable piece that clips into the frame piece. Its purpose is to keep the subject's mouth open. It's not necessary to know precisely where the untreated jaw is, all that is needed is to make sure that the subject is not able to bite the robot. Thus, the jaw plate may have one of more forked structures, which extend from it and towards the opposite jaw, and which slot over a corresponding fixture attached to the other jaw, e.g. on the frame or extension arm. The corresponding fixture may be as shown in figure 8, where a flat blade-like structure 322 is configured to go into the fork-like structure 702 to form a jaw lock. The jaw plate may also comprise one or more C-shaped protrusions for clipping to a suction cannula.

The fork-like structures can clear the blade-like structures so long as the mouth of the subject is sufficiently open. Therefore, the jaw plate may be inserted after the main body of the dental splint is placed inside the subject's mouth. Once the jaw plate is in place, the subject can then shut their mouth to a point and the fork-like structures will engage the blade-like structures. It is then not possible for the subject to shut their mouth any further. The blade-like structures and fork-like structures may also comprise shaped mating surfaces which resist movement apart once engaged together.

The dental splint may be made specific to an individual subject. Upon imaging the teeth of the subject, the frame, bridge, one or more pairs of clamping elements, and jaw plate may be formed to match the shape of the subject's teeth and surrounding structures. For example, the clamping elements and the jaw plate may be made to fit the shape of the tooth or teeth of the upper or lower jaw. The bridge may match the contours of the hard palate of the subject.

The dental splint may be arranged so that during use it does not impede access to the teeth on which a procedure is to be performed.

The dental splint may be manufactured so as to match the dentition of a specific subject. The dental splint may be formed in dependence on a three-dimensional scan of the subject's dentition. This can result in improved fit between the splint and the teeth of the subject, and hence reduced motion of the splint relative to the teeth of the subject when the splint is attached in place. The dental splint may be formed by additive manufacturing, e.g. three-dimensional printing, or by selective removal of material from a pre-formed blank.

There is provided a method for providing a positional reference with respect to dentition of a subject. The method comprises causing a dental splint as described herein to grip a tooth of the subject and detecting a reference unit carried by the or each extension arm of the dental splint. The method may comprise forming the first and second clamping elements to match the contours of the first tooth of the subject or forming the third and fourth clamping elements to match the contours of the second tooth of the subject. That is, the method may comprise producing the clamping elements to be subject specific. This may comprise producing the subject specific parts of the splint based on 3D images of the subject's teeth. For example, this may comprise forming moulds for parts of the splint or the splint itself. The processes may comprise casting, moulding, etching, or 3D printing.

Therefore, the method may comprise scanning the first tooth or second tooth so as to form a set of three-dimensional data representing the shape of the first tooth or second tooth. Then forming the first and second clamping elements or the third and fourth clamping elements in dependence on the set of three-dimensional data.

The method may comprise forming the first and second clamping elements or the third and fourth clamping element by additive manufacturing. Additive manufacturing is a process that creates a three-dimensional object from a digital model by adding material in layers. An example of additive manufacturing is 3D printing. Additive manufacturing can be used with lots of different materials such as polymers, metals, alloys, and ceramics. The additive manufacturing may be used to make the subject specific parts of the splint, or moulds for the subject specific parts.

Figure 9 shows an example of the dental splint 100 viewed from above. The dental splint may comprise one or more stopper arrangements 910 spanning the dental arch of the frame 102. In this example there are two stoppers 910a and 910b. Each stopper may comprise two bumpers with a break or gap 902 in between. The stopper 910 may act as a pair of bumpers, where the ends of the bumpers either side of the gap 902 are configured to meet when pressure is applied to outside of the frame 102. Such pressure may be required to enable the frame to sit within the roof of the subject's mouth. However, the frame may break if too much pressure is applied. Therefore, the stopper in the configuration of a pair of bumpers may limit the minimum distance between the pairs of clamping elements and prevent over-bending and breakage of the frame. Thus, the frame can flex while being put into place without snapping in the middle.

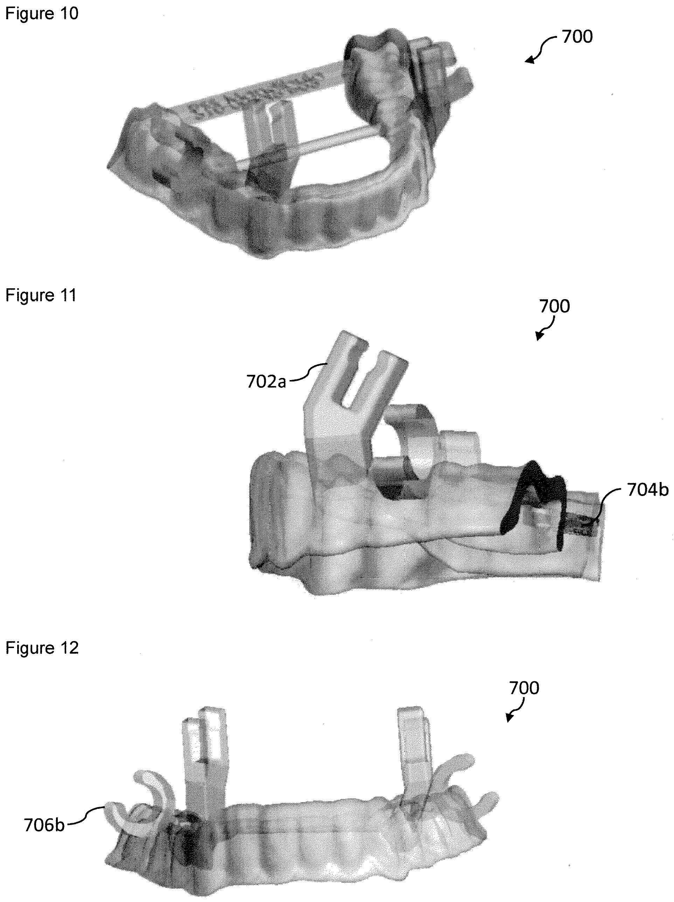

Figure 10 shows a perspective view of an example jaw plate 700.

Figure 11 shows a side on perspective view of an example jaw plate 700 including an end region/cross-section near the tongue restraint 704b. An example recessed shape of the formation 702a for engaging the frame 102 is also shown. The end region/cross-section illustrates that the jaw plate 700 may also be shaped to match the contours of the teeth of the subject. The jaw plate 700 may be shaped to match the contours of all of the teeth in a single dental arch of the subject. The jaw plate may be shaped to match the contours of molars and/or premolars of the subject. The jaw plate 700 may be shaped to match the contours of some or all of the teeth on the lower dental arch of the subject.

Figure 12 shows a front view of an example jaw plate 700. An example recessed shape of the formation 706b for holding a suction tube is also shown. As the suction tube is typically cylindrical in shape, the recess may be circular or annular with an arc section missing for accepting the suction tube.

Figure 13 shows an example dental splint 100. In this example the dental splint comprises one extension arm 108 which is rigidly fixed to a frame 102 configured to mechanically grip at least a first tooth of a subject and extending from the frame for supporting a reference unit. There is also shown an example of a corresponding protrusion 602 for engaging the shoulder 504 on the locking piece 500.

Figure 14 shows a rear view of an example dental splint 100 according to the above description.

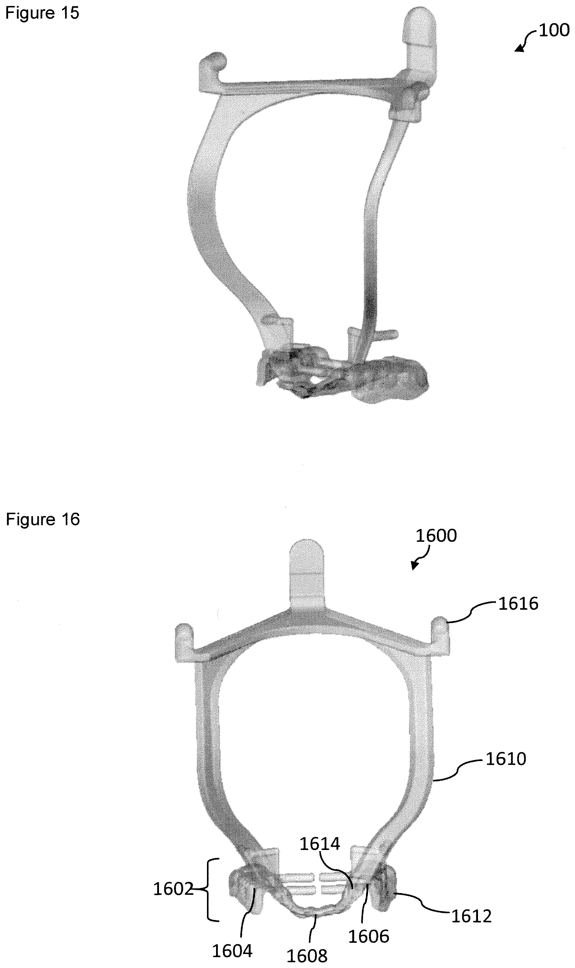

Figure 15 shows a perspective view of an example dental splint 100.

Figure 16 shows a perspective view of an example of another embodiment of a dental splint 1600 viewed from the front. There is provided a further dental splint 1600. The dental splint comprising an arcuate jaw clamp 1602 extending around an arc of greater than 120 degrees and having a first recess 1604 at one end thereof for receiving a first molar or premolar of a subject, and a second recess 1606 at the other end thereof for receiving a second molar or premolar of the subject. The first and second molars or premolars being in the same dental arch but different quadrants thereof. The jaw clamp comprising a bridge 1608 extending between the first recess 1604 and the second recess 1606 and shaped such that when the first recess receives the first molar or premolar and the second recess receives the second molar or premolar, the bridge 1608 does not occlude the facial surfaces of the incisors of the said dental arch. That is, the bridge is an integral part of the arcuate jaw clamp between the first and second recesses.

Each recess may comprise a first clamping element 1612 and a second clamping element 1614. The clamping elements of each recess may be moveable with respect to each other to mechanically grip a tooth of the subject located in the respective recess.

The splint may comprise at least one extension arm 1610 rigidly attached to the arcuate jaw clamp 1602 and extending from the arcuate jaw clamp for supporting a reference unit at a location outside the oral cavity of the subject when the arcuate jaw clamp is engaged with one or more teeth in the recesses.

The extension arm may carry a receptacle 1616 for releasably receiving the reference unit. The extension arm may carry the reference unit.

It should be understood that additional features as described above in relation to the embodiments show in figures 1 to 15 may also be implemented alongside the features described in reference to the embodiment show and described in reference figure 16.

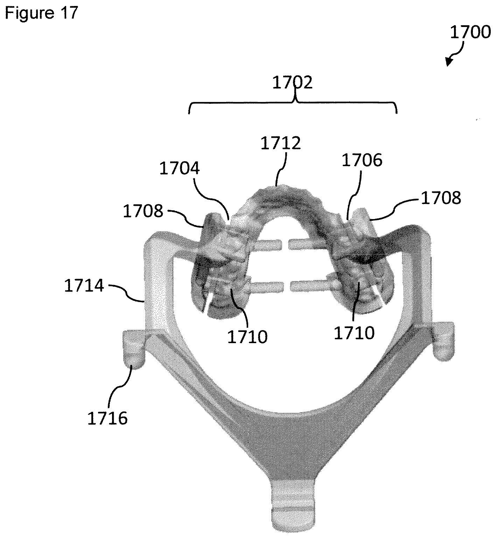

Figure 17 shows a perspective view of an example of another embodiment of a dental splint 1700 viewed from below. There is provided another embodiment of the dental splint. The dental splint 1700 comprises an arcuate jaw clamp 1702 extending around an arc of greater than 120 degrees and having a first recess 1704 at one end thereof and a second recess 1706 at the other end thereof. Each recess has at least one side wall for contacting a lateral surface of at least one tooth of a subject and a basal surface extending transversely to the side wall for contacting occlusal surfaces of at least one tooth in the recess for limiting the insertion of at least said tooth into the recess in a first direction. Both the basal surfaces lie on a common basal plane and the jaw clamp has a bridge 1712 extending between the first recess and the second recess, a central region of the bridge extending, on the convex side of the arc, no further in a direction opposite to the first direction than the basal plane.

Each recess may comprise a first clamping element 1708 and a second clamping element 1710, the clamping elements of each recess being moveable with respect to each other to mechanically grip a tooth of the subject located in the respective recess.

The splint may comprise at least one extension arm 1714 rigidly attached to the arcuate jaw clamp and extending from the arcuate jaw clamp for supporting a reference unit at a location outside the oral cavity of the subject when the arcuate jaw clamp is engaged with one or more teeth in the recesses.

The extension arm may carry a receptacle 1716 for releasably receiving the reference unit. The extension arm may carry the optical reference unit.

It should be understood that additional features as described above in relation to the embodiments show in figures 1 to 15 may also be implemented alongside the features described in reference to the embodiment show and described in reference figure 17.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description it will be evident to a person skilled in the art that various modifications may be made within the scope of the invention.

## Claims

1. A dental splint for providing a positional reference with respect to dentition of a subject, the splint comprising:
a frame having a first clamping element and a second clamping element movable with respect to the first clamping element to mechanically grip a first tooth of the subject; and
a first extension arm rigidly attached to the frame and extending from the frame for supporting a reference unit at a location outside the oral cavity of the subject when the frame is gripping the first tooth.

2. A dental splint as claimed in claim 1, wherein the first clamping element is integral with the second clamping element, and the first and second clamping elements are flexibly interconnected such that the first and second clamping elements can be urged towards each other to grip the first tooth.

3. A dental splint as claimed in claim 2, the splint comprising a locking piece sized to be engageable against the first and second clamping elements to urge the first and second clamping elements into contact with the first tooth.

4. A dental splint as claimed in claim 3, wherein the locking piece defines a channel having side walls that can be engaged against the first and second clamping elements to urge the first and second clamping elements into contact with the first tooth.

5. A dental splint as claimed in claim 3 or 4, wherein the locking piece is configured to releasably latch to the first and second clamping elements for holding it in engagement with the first and second clamping elements.

6. A dental splint as claimed in any of claims 3 to 5, wherein the locking piece is non-integral with the clamping elements.

7. A dental splint as claimed in any of claims 3 to 6, wherein the locking piece comprises a shoulder configured to engage a complementary protrusion located elsewhere on the dental splint for retaining the locking piece on the dental splint when not latched to the first and second clamping elements.

8. A dental splint as claimed in any preceding claim, the frame having a third clamping element and a fourth clamping element movable with respect to the third clamping element to mechanically grip a second tooth of the subject; the second tooth being on the opposite quadrant of the same jaw to the first tooth.

9. A dental splint as claimed in claim 8, the splint comprising a second locking piece sized to be engageable against the third and fourth clamping elements to urge the third and fourth clamping elements into contact with the second tooth.

10. A dental splint as claimed in claim 8 or 9, wherein the first extension arm is attached to the frame adjacent the first and second clamping elements and the splint comprises a second extension arm for supporting the reference unit at a location outside the oral cavity of the subject when the frame is gripping the second tooth, the second extension arm being rigidly attached to the frame adjacent the third and fourth clamping elements.

11. A dental splint as claimed in any of claims 8 to 10, wherein:
one of the first and second clamping elements has a leading end and a trailing end, the trailing end being cupped so as to engage the occlusal surface of the first tooth and thereby limit insertion of the first tooth between the first and second clamping elements;
one of the third and fourth clamping elements has a further leading end and a further trailing end, the further trailing end being cupped so as to engage the occlusal surface of the second tooth and thereby limit insertion of the second tooth between the third and fourth clamping elements;
the leading end and the further leading end lie in a common planar zone; and
the frame comprises a bridge extending between (i) the first and second and (ii) the third and fourth clamping elements, the bridge being located no closer to the cupped regions of the first to fourth clamping elements than the common planar zone.

12. A dental splint as claimed in any preceding claim, wherein the or each extension arm carries a receptacle for releasably receiving the reference unit.

13. A dental splint as claimed in any of claims 1 to 11, wherein the or each extension arm carries the reference unit.

14. A dental splint as claimed in any preceding claim, wherein the reference unit is an optical reference unit comprising a plurality of optical reference sites.

15. A dental splint as claimed in claim 14, wherein each optical reference site comprises a recess for releasably receiving a reflector and a key surface shaped for fixing the position of the reflector relative to the frame.

16. A dental splint as claimed in claim 14 or 15, wherein the optical reference unit has at least three optical reference sites, the optical reference sites being located so as to be coplanar and not all collinear.

17. A dental splint as claimed in any of claims 1 to 13, wherein the reference unit is an inertial reference unit or a magnetic reference unit.

18. A dental splint as claimed in any preceding claim, the splint being arranged so that when the first tooth or the second tooth is a molar or premolar of the subject, the splint does not occlude the facial surfaces of the incisors of the same dental arch as the first tooth.

19. A dental splint as claimed in any preceding claim, wherein the first and second clamping elements, and the third and fourth clamping elements are shaped to match the contours of molars and/or premolars of a subject.

20. A dental splint as claimed in any preceding claim, wherein the first tooth or the second tooth is of a first dental arch of the subject and the splint comprises a jaw plate for engaging the other dental arch of the subject, the jaw plate being releasably attachable to the frame in a position to engage the other dental arch and thereby restrict motion of the jaws of the subject towards each other.

21. A dental splint as claimed in claim 20, wherein either the frame or the jaw plate comprises a tongue restraint for restraining the tongue of the subject.

22. A dental splint as claimed in claim 20 or 21, wherein either the frame or the jaw plate comprises a formation for holding a suction tube in the subject's oral cavity.

23. A method for providing a positional reference with respect to dentition of a subject, the method comprising:
causing a dental splint as claimed in any preceding claim to grip a tooth of the subject; and
imaging a reference unit carried by the or each extension arm of the dental splint.

24. A method as claimed in claim 23, the method comprising:
forming the first and second clamping elements to match the contours of the first tooth of the subject or forming the third and fourth clamping elements to match the contours of the second tooth of the subject.

25. A method as claimed in claim 24, the method comprising:
scanning the first tooth or second tooth so as to form a set of three-dimensional data representing the shape of the first tooth or second tooth; and
forming the first and second clamping elements or the third and fourth clamping elements in dependence on the set of three-dimensional data.

26. A method as claimed in claim 25, comprising forming the first and second clamping elements or the third and fourth clamping element by additive manufacturing.

27. A dental splint comprising:
an arcuate jaw clamp extending around an arc of greater than 120 degrees and having a first recess at one end thereof and a second recess at the other end thereof, each recess having at least one side wall for contacting a lateral surface of at least one tooth of a subject and a basal surface extending transversely to the side wall for contacting occlusal surfaces of at least one tooth in the recess for limiting the insertion of at least said tooth into the recess in a first direction;
wherein both the basal surfaces lie on a common basal plane and the jaw clamp has a bridge extending between the first recess and the second recess, a central region of the bridge extending, on the convex side of the arc, no further in a direction opposite to the first direction than the basal plane.

28. A dental splint as claimed in claim 27, wherein each recess comprises a first clamping element and a second clamping element, the clamping elements of each recess being moveable with respect to each other to mechanically grip a tooth of the subject located in the respective recess.

29. A dental splint as claimed in claim 27 or 28, wherein the splint comprises at least one extension arm rigidly attached to the arcuate jaw clamp and extending from the arcuate jaw clamp for supporting a reference unit at a location outside the oral cavity of the subject when the arcuate jaw clamp is engaged with one or more teeth in the recesses.

30. A dental splint as claimed in claim 29, wherein the extension arm carries a receptacle for releasably receiving the reference unit.

31. A dental splint as claimed in claim 29, wherein the extension arm carries the reference unit.

32. A dental splint comprising:
an arcuate jaw clamp extending around an arc of greater than 120 degrees and having a first recess at one end thereof for receiving a first molar or premolar of a subject, and a second recess at the other end thereof for receiving a second molar or premolar of the subject, the first and second molars or premolars being in the same dental arch but different quadrants thereof;
the jaw clamp comprising a bridge extending between the first recess and the second recess and shaped such that when the first recess receives the first molar or premolar and the second recess receives the second molar or premolar, the bridge does not occlude the facial surfaces of the incisors of the said dental arch.

33. A dental splint as claimed in claim 32, wherein each recess comprises a first clamping element and a second clamping element, the clamping elements of each recess being moveable with respect to each other to mechanically grip a tooth of the subject located in the respective recess.

34. A dental splint as claimed in claim 32 or 33, wherein the splint comprises at least one extension arm rigidly attached to the arcuate jaw clamp and extending from the arcuate jaw clamp for supporting a reference unit at a location outside the oral cavity of the subject when the arcuate jaw clamp is engaged with one or more teeth in the recesses.

35. A dental splint as claimed in claim 34, wherein the extension arm carries a receptacle for releasably receiving the reference unit.

36. A dental splint as claimed in claim 34, wherein the extension arm carries the reference unit.
